# EUROPÄISCHE PATENTSCHRIFT

(11) **EP 4 187 103 B1**
(45) Veröffentlichungstag und Bekanntmachung des Hinweises auf die Patenterteilung: **30.07.2025**
(21) Anmeldenummer: 22206659.9
(22) Anmeldetag: 10.11.2022
(51) Int. Cl.: A61M 16/00, A61M 16/01, A61M 16/10, A61M 16/12, F04D 29/42, F04D 29/10, F04D 29/58

(54) **KONTAMINATIONSVERMEIDUNG IM GEBLÄSE VON BEATMUNGSGERÄTEN**
CONTAMINATION PREVENTION IN VENTILATOR FAN
PRÉVENTION DE CONTAMINATION DANS UN VENTILATEUR D'APPAREIL RESPIRATOIRE

(30) Priorität: 29.11.2021 DE 102021005886
(43) Veröffentlichungstag der Anmeldung: 31.05.2023
(73) Patentinhaber: Löwenstein Medical Technology S.A., 2557 Luxembourg (LU)
(72) Erfinder: Schregel, Christian-Georg, 61169 Friedberg (DE)
(74) Vertreter: Löwenstein Medical IP

(56) Entgegenhaltungen:
- FR-A1- 2 910 081
- FR-B1- 2 910 078
- US-A1- 2010 189 554

## Beschreibung

Beatmungs-, Anästhesie- und Atemtherapiegeräte zur Beatmung bzw. Atmungsunterstützung oder zur Hustenunterstützung weisen für die Durchführung einer Atemtherapie ein Gebläse zum Erzeugen einer Atemluftströmung auf. Im Gehäuse des Gebläses ist üblicherweise wenigstens ein drehbar gelagertes Lüfterrad (auch als Laufrad oder Impeller bezeichnet) mit einer Mehrzahl von Schaufelelementen angeordnet, das von einem Motor angetrieben wird.

Insbesondere beim Betrieb eines Gebläses im Rahmen eines halbgeschlossenen Kreislaufs, wie es z. B. bei Anästhesiegeräten der Fall ist, kann es durch die Atemluft des Patienten auch an schlecht erreichbaren Stellen, wie z. B. an der Antriebswelle oder im Motorlager des Gebläses, zu einer Verkeimung kommen.

Bei der Aufbereitung von Gebläsen im Reinigungs- und Desinfektionsgerät treten zwei grundlegende Probleme auf. Das erste Problem sind die Auswirkungen der Reinigung bzw. Desinfektion auf den Motor. Kontakt mit den im Allgemeinen chemisch aggressiven Reinigungsflüssigkeiten führt im Motor u. a. zu Verschleiß an Lagern, angegriffenen Klebestellen oder bei entsprechend ungünstigen Materialpaarungen sogar zu Kontaktkorrosion. Das zweite Problem ist die schlechte Reinigungsleistung der schlecht erreichbaren Stellen. Im Vergleich zu den direkt zugänglichen Teilen wie dem Lüfterrad oder dem Lüfterradgehäuse kann die Reinigungsflüssigkeit nur indirekt in die Räume der schlecht erreichbaren Stellen gelangen, was die Aufprallenergie der Flüssigkeit mindert und potenziell auch eine Temperatursenke darstellt.

Eine rein mechanische Abdichtung des Lüfterradgehäuses zur Vermeidung eines Eindringens von Keimen ist in der Intensivbeatmung und Anästhesie nachteilig, da Gebläse dort mit sehr hohen Drehzahlen (50.000 bis 100.000 Umdrehungen pro Minute) betrieben werden müssen und vollständig abdichtende Lager mit höheren Reibungsverlusten behaftet sind.

US 2010/189554 A1 und FR 2 910 081 A1 offenbaren ein Gebläse für ein Beatmungsgerät. Das Gebläse umfasst einen Motor, der in einem Motorteil angeordnet ist, einen Motorvorhof und einen Gebläsekopf, in dem ein vom Motor angetriebenes Lüfterrad zum Erzeugen einer Luftströmung angeordnet ist. Im Motorteil ist zusätzlich ein zweites Lüfterrad angeordnet, das einen Luftstrom zum Kühlen des Motors erzeugen kann.

FR 2 910 078 B1 offenbart ein ähnliches Gebläse, bei dem zwischen dem Motorvorhof und dem Gebläsekopf zusätzlich ein Sammelbehälter ausgebildet ist, in dem aus dem Gebläsekopf austretender Sauerstoff und aus dem Motorvorhof austretende Luft gesammelt werden können.

Aufgabe der vorliegenden Erfindung ist, ein effizientes, langlebiges Beatmungsgerät zur Verfügung zu stellen, welches keimfrei betrieben werden kann.

Diese Aufgabe wird gelöst mit einem Beatmungsgerät des Anspruchs 1. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die Erfindung betrifft ein Beatmungsgerät mit wenigstens einem Motor betriebenen Gebläse zum Erzeugen einer Luftströmung. Erfindungsgemäß ist das Gebläse eingerichtet und ausgebildet, dass während der Erzeugung der Luftströmung im Gebläse unterschiedliche Druckbereiche mit unterschiedlichen Drücken derart ausgebildet werden, dass eine Luftströmung in Richtung des Motors verhindert wird.

Erfindungsgemäß ist das Beatmungsgerät so ausgebildet, dass das Gebläse mindestens ein Motorteil mit dem Motor und einer Antriebswelle sowie einen Gebläsekopf mit einem drehbar gelagerten Lüfterrad umfasst, wobei zwischen dem Motorteil und dem Gebläsekopf ein Motorvorhof angeordnet ist, durch den die Antriebswelle des Motors verläuft.

In manchen Ausführungsformen ist das Beatmungsgerät so ausgebildet, dass im Gebläsekopf mindestens ein Druckbereich mit einem Druck anliegt und im Motorvorhof mindestens ein Druckbereich mit einem Druck anliegt, wobei der Druck im Motorvorhof gleich dem Druck im Gebläsekopf ist.

In manchen Ausführungsformen ist das Beatmungsgerät so ausgebildet, dass im Gebläsekopf mindestens ein Druckbereich mit einem Druck anliegt und im Motorvorhof mindestens ein Druckbereich mit einem Druck anliegt, wobei der Druck im Motorvorhof größer als der Druck im Gebläsekopf ist.

In manchen Ausführungsformen ist das Beatmungsgerät so ausgebildet, dass der Druck im Motorvorhof konstant ist oder dynamisch an den Druck im Gebläsekopf angepasst wird.

In manchen Ausführungsformen ist das Beatmungsgerät so ausgebildet, dass der Druck im Motorvorhof durch eine Beaufschlagung mit dem Druck und/oder durch eine weitere Strömung erzeugt wird.

Erfindungsgemäß ist das Beatmungsgerät so ausgebildet, dass das Gebläse mindestens eine Zuführung umfasst, wobei durch die Zuführung ein Fluid, insbesondere ein Atemgas oder ein Atemgasgemisch, in den Motorvorhof geleitet wird.

Erfindungsgemäß ist zwischen dem Motorvorhof und dem Gebläsekopf eine Durchführung eingerichtet und angeordnet, wobei die Antriebswelle vom Motor durch den Motorvorhof und die Durchführung zum Lüfterrad im Gebläsekopf führt.

Erfindungsgemäß ist der Motorvorhof im Wesentlichen luftdicht mit dem Gebläsekopf verbunden, wobei lediglich die Durchführung eine Leckage zwischen dem Motorvorhof und dem Gebläsekopf zulässt.

In manchen Ausführungsformen umfasst das Gebläse einen Saugstutzen mit einer Einlassöffnung und einen Druckstutzen mit einer Auslassöffnung, wobei die Luftströmung im Gebläsekopf erzeugt wird und von der Einlassöffnung zu der Auslassöffnung strömt. Die unterschiedlichen Druckbereiche sind derart ausgebildet, dass im Gebläse mindestens eine zusätzliche Strömung erzeugt wird, wobei die zusätzliche Strömung vom Motorvorhof durch die Durchführung in den Gebläsekopf strömt.

In manchen Ausführungsformen ist das Beatmungsgerät so ausgebildet, dass im Gebläsekopf die Luftströmung erzeugt wird, die von einer Einlassöffnung zu einer Auslassöffnung strömt, und eine zusätzliche Strömung erzeugt wird.

In manchen Ausführungsformen ist das Beatmungsgerät so ausgebildet, dass der Druck im Motorvorhof größer ist als der Druck im Gebläsekopf, sodass die Strömung durch die Durchführung vom Motorvorhof in den Gebläsekopf strömt.

In manchen Ausführungsformen ist das Beatmungsgerät so ausgebildet, dass der Motorvorhof durch die zusätzliche Strömung als Reinraumschleuse fungiert, wobei ein Eindringen von Keimen vom Gebläsekopf in den Motorvorhof verhindert wird.

In manchen Ausführungsformen ist das Beatmungsgerät so ausgebildet, dass der Motor durch die zusätzliche Strömung keimfrei betrieben wird.

Erfindungsgemäß ist das Beatmungsgerät so ausgebildet, dass der Motorvorhof über die Zuführung mit dem Druck P2 beaufschlagt wird.

In manchen Ausführungsformen umfasst das Gebläse mindestens eine Abführung, wobei durch die Abführung ein Fluid, insbesondere ein Atemgas oder ein Atemgasgemisch, aus dem Motorvorhof abgeleitet wird, wobei die Abführung am Motorvorhof und/oder am Motorteil angeordnet ist.

In manchen Ausführungsformen ist das Beatmungsgerät so ausgebildet, dass der Motorvorhof über die weitere Strömung mit dem Druck P2 beaufschlagt wird, wobei die Zuführung und die Abführung eingerichtet und ausgebildet sind, um die weitere Strömung zu erzeugen, indem ein Fluid über die Zuführung in den Motorvorhof zugeführt wird und über die Abführung aus dem Motorvorhof abgeleitet wird.

In manchen Ausführungsformen ist die Zuführung am Motorvorhof und/oder am Motorteil angeordnet.

In manchen Ausführungsformen ist das Beatmungsgerät so ausgebildet, dass die Zuführung am Motorvorhof angeordnet ist und der Motorvorhof über die Zuführung mit dem Druck beaufschlagt wird.

In manchen Ausführungsformen ist die Zuführung am Motorteil angeordnet, wobei das Motorteil mindestens einen Kanal aufweist, an den die Zuführung luftdicht gekoppelt ist, und wobei der Kanal in den Motorvorhof mündet.

In manchen Ausführungsformen ist das Beatmungsgerät so ausgebildet, dass der Motorvorhof über die Zuführung und den Kanal mit dem Druck beaufschlagt wird und/oder der Druck durch die weitere Strömung erzeugt wird.

In manchen Ausführungsformen ist das Beatmungsgerät so ausgebildet, dass der Motorvorhof eine Abführung umfasst und die weitere Strömung von der Zuführung durch den Kanal in den Motorvorhof und von dort über die Abführung strömt.

In manchen Ausführungsformen ist das Beatmungsgerät so ausgebildet, dass die weitere Strömung derart durch den mindestens einen Kanal geleitet wird, dass die weitere Strömung den Motor kühlt.

In manchen Ausführungsformen ist das Gebläse einteilig oder zweiteilig ausgebildet.

In manchen Ausführungsformen ist der Motorvorhof eingerichtet und ausgebildet ist, um eine Trennung von Gebläsekopf und Motorteil zuzulassen.

In den Figuren sind Ausführungsbeispiele des erfindungsgemäßen Beatmungsgerätes dargestellt. Es zeigen:
- **Fig. 1**: einen schematischen Ausschnitt aus einem erfindungsgemäßen Beatmungsgerät mit einem Gebläse im Querschnitt;
- **Fig. 2**: einen schematischen Ausschnitt aus dem erfindungsgemäßen Beatmungsgerät mit dem Gebläse im Querschnitt mit Darstellung verschiedener Strömungen und unterschiedlicher Druckbereiche;
- **Fig. 3**: einen schematischen Ausschnitt aus einem alternativen Ausführungsbeispiel des erfindungsgemäßen Beatmungsgerätes mit dem Gebläse im Querschnitt;
- **Fig. 4**: einen schematischen Ausschnitt aus einem weiteren Ausführungsbeispiel des erfindungsgemäßen Beatmungsgerätes mit dem Gebläse im Querschnitt.

In den nachfolgenden Ausführungsbeispielen ist ein erfindungsgemäßes Beatmungsgerät beschrieben. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

Unter einem Beatmungsgerät sind im Sinne der Erfindung sämtliche Geräte zu verstehen, die einen Patienten oder sonstigen Anwender bei der natürlichen Atmung unterstützen und/oder die Beatmung eines Anwenders oder Patienten übernehmen und/oder einer Atemtherapie dienen und/oder anderweitig auf die Atmung eines Anwenders oder Patienten einwirken. Darunter fallen zum Beispiel, aber nicht ausschließlich, Beatmungsgeräte für klinische oder Heimanwendungen, Atemtherapiegeräte, CPAP-, APAP- sowie BiLevel-Geräte, Highflow-Therapiegeräte, Narkose- bzw. Anästhesiegeräte, klinische, außerklinische oder Notfall-Beatmungsgeräte, sauerstoffliefernde Geräte, Diagnosesysteme sowie Hustentherapiegeräte bzw. Hustenmaschinen.

Das Beatmungsgerät 1 ist mit einem im Geräteinneren untergebrachten Gebläse 10 (auch als Blower bezeichnet) ausgestattet, das mindestens ein Lüfterrad 24 umfasst, mit dem eine Luftströmung S für eine Beatmung oder Atemtherapie erzeugt wird. Insbesondere wird eine Atemluftströmung S für eine Beatmung oder Atemtherapie erzeugt. Atemluft umfasst im Sinne der Erfindung jegliches Fluid, Atemgas und/oder Gasgemisch, welches sich zur Beatmung, Atmung und/oder Atemtherapie eignet und eingesetzt werden kann. Atemluft und Frischgas werden hierin als Synonyme verwendet. Die Atemluft bzw. das Frischgas kann auch Sauerstoff oder mit Sauerstoff angereicherte Luft sein. Insbesondere kann die Atemluft bzw. das Frischgas auch mindestens ein Narkosegas enthalten und für Anästhesiegeräte geeignet sein.

Das Gebläse 10 wird über eine im Geräteinneren angeordnete Steuereinrichtung angesteuert. Beispielsweise stellt die Steuereinrichtung in Abhängigkeit von Therapievorgaben eine bestimmte Drehzahl des Lüfterrades 24 ein beziehungsweise regelt die Lüfterdrehzahl auf einen Sollwert.

Das Beatmungsgerät 1 weist eine Schnittstelle zum Koppeln eines Schlauchsystems auf, über das dem Patienten oder Anwender eine (Atem-)Luftströmung S zur Beatmung, Atemunterstützung bzw. Hustenunterstützung zugeführt werden kann. Zu diesem Zwecke kann an das Schlauchsystem ein Patienteninterface angeschlossen werden. Als Patienteninterface ist im Sinne der Erfindung jegliches Peripheriegerät zu verstehen, welches zur Interaktion mit einem Lebewesen ausgebildet ist. Insbesondere ist das Patienteninterface zu Therapie- und/oder Diagnosezwecken in Verbindung mit dem Beatmungsgerät 1 ausgebildet. Das Patienteninterface kann als Atemmaske ausgebildet sein. Darunter fallen zum Beispiel, aber nicht ausschließlich, Nasenmasken, Nasenpolstermasken, Nasenbrillen bzw. Sauerstoffbrillen, Full-Face- oder Totalface-Masken sowie Trachealtuben bzw. -kanülen.

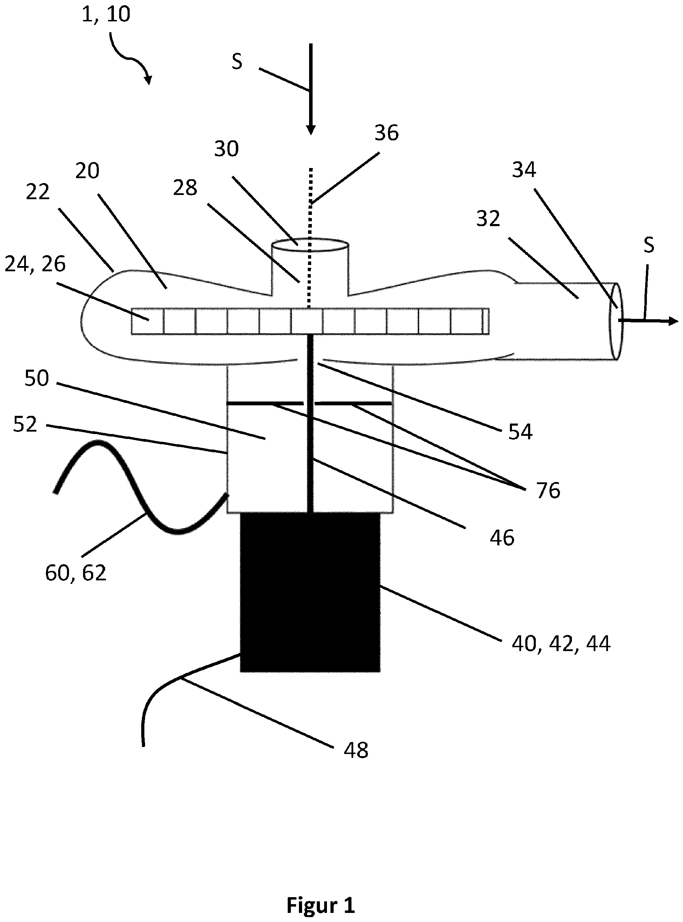

**Fig. 1** zeigt einen schematischen Ausschnitt aus dem Beatmungsgerät 1 mit dem Gebläse 10 im Querschnitt. Das Gebläse 10 umfasst einen Gebläsekopf 20, ein Motorteil 40 und einen Motorvorhof 50.

Das Motorteil 40 hat ein Motorgehäuse 42. In dem Motorgehäuse 42 ist eine Antriebsvorrichtung bzw. ein Motor 44 mit einem elektrischen Antrieb gelagert. Um den Motor 44 zu betreiben, kann das Motorteil 40 einen elektrischen Anschluss 48 aufweisen, der den Motor 44 mit Energie versorgen kann. Der Motor 44 ist mir einer Antriebswelle 46 verbunden, die über den Motor 44 in eine Rotation gebracht werden kann. Die Antriebswelle 46 kann einteilig oder zweiteilig ausgeführt sein. Der Motor 44 kann über eine Steuereinrichtung des Beatmungsgerätes 1 angesteuert werden, sodass bestimmte Drehzahlen dynamisch eingestellt werden können.

Der Gebläsekopf 20 hat ein Lüfterradgehäuse 22, in dem mindestens ein Lüfterrad 24 angeordnet ist. Das Lüfterrad 24 ist in dem Lüfterradgehäuse 22 drehbar gelagert angeordnet. Das Lüfterrad 24 umfasst mehrere Schaufelelemente 26. Zum Drehen des Lüfterrades 24 weist das Gebläse 10 im Motorteil 40 den Motor 44 mit einem elektrischen Antrieb auf. Der Motor 44 kann über die Antriebswelle 46 Rotationsenergie auf das Lüfterrad 24 übertragen. Durch die Rotationsbewegung entsteht am Lüfterrad 24 eine Druck- und eine Unterdruckseite, wodurch die (Atem-)Luftströmung S mit einer Strömungsrichtung entsteht. Die Strömungsrichtung ist in den Figuren mit Pfeilen gekennzeichnet. Die Luftströmung S verläuft im Wesentlichen von einer Einlassöffnung 30 zu einer Auslassöffnung 34 des Gebläses 1. Die Steuereinrichtung des Beatmungsgerätes 1 ist eingerichtet und ausgebildet, das Lüfterrad 24 dynamisch anzusteuern und mit hohen Drehzahlen bis zu 100.000 Umdrehungen pro Minute zu betreiben.

Das Lüfterradgehäuse 22 des Gebläsekopfes 20 ist in der Regel spiralförmig. Das Gebläse 10 umfasst einen Saugstutzen 28 mit der Einlassöffnung 30. Der Saugstutzen 28 weist eine Mittelachse 36 auf. Gebläse 10, die in Beatmungsgeräten 1 eingesetzt werden, sind üblicherweise Radialgebläse. Bei Radialgebläsen tritt ein Fluid, beispielsweise Atemluft, in Achsrichtung in den Gebläsekopf 20 ein und tritt senkrecht zur Achsrichtung wieder aus dem Gebläse 10 heraus. Das Gebläse 10 kann neben Atemluft auch jedes andere Gasgemisch transportieren, das für eine Atemtherapie benötigt wird. Über den Saugstutzen 28 kann das Fluid, beispielsweise Atemluft, angesaugt werden. Die Atemluft tritt durch den Saugstutzen 28 in Achsrichtung in den Gebläsekopf 20 ein. Das Gebläse 10 umfasst weiterhin einen Druckstutzen 32 mit der Auslassöffnung 34. Über den Druckstutzen 32 kann das Fluid abgegeben werden. Die Atemluft tritt durch den Druckstutzen 32 senkrecht zur Achsrichtung wieder aus dem Gebläsekopf 20 heraus. Das spiralförmige Lüfterradgehäuse 22 sorgt dafür, dass die Atemluft im Gebläsekopf 20 gesammelt und zum Druckstutzen 32 geleitet wird, wo sie aus dem Gebläsekopf 20 austritt. Dabei wird verhindert, dass kreisende Austrittsströmungen entstehen, die zu Verlusten führen. Gleichzeitig wird durch das Spiralgehäuse 10 ein Teil der Geschwindigkeitsenergie der Atemluft in Druckenergie umgewandelt.

An den Saugstutzen 28 und/oder den Druckstutzen 32 können Schlauchverbindungen angeschlossen werden (nicht gezeigt). Über eine Schlauchverbindung am Saugstutzen 28 kann beispielsweise Frischgas in das Lüfterradgehäuse 22 hineingeführt werden. Über eine Schlauchverbindung am Druckstutzen 32 kann das Frischgas dem Patienten zugeführt werden.

Saugstutzen 28 und/oder Druckstutzen 32 können zudem dafür genutzt werden, das Gebläse zu reinigen und/oder zu desinfizieren. Die Reinigung und/oder Desinfektion kann beispielsweise derart vollzogen werden, dass das Gebläse 1 in ein Reinigungs- bzw. Desinfektionsgerät gegeben wird. Sodann kann über den Druckstutzen 32 und/oder den Saugstutzen 28 eine Reinigungsflüssigkeit in das Lüfterradgehäuse 22 eingeleitet werden. Als Reinigungsflüssigkeit wird hierin jegliches Mittel bezeichnet, dass zur Reinigung und/oder Desinfektion des Gebläses 1 geeignet ist. Reinigung und Desinfektion werden hierin gleichbedeutend verwendet. Eine Reinigung kann eine Desinfektion mit einschließen und eine Desinfektion kann eine Reinigung mit einschließen.

Beispielsweise können an den Druckstutzen 32 und/oder an den Saugstutzen Schlauchverbindungen angeschlossen werden, die das Gebläse 1 mit dem Reinigungs- bzw. Desinfektionsgerät verbinden. Die Reinigungsflüssigkeit kann beispielsweise mit mindestens 50 mbar, bevorzugt mit mindestens 80 mbar, besonders bevorzugt mit mindestens 100 mbar, in das Lüfterradgehäuse 22 eingeleitet werden. Der Druck, mit dem die Reinigungsflüssigkeit eingeleitet wird, sollte so hoch gewählt werden, dass die Reinigungsflüssigkeit möglichst alle Bereiche innerhalb des Gebläsekopfes 20 erreicht. Gleichzeitig sollte der Druck so gering gewählt werden, dass das Gebläse 1 nicht beschädigt wird.

Für eine Reinigung bzw. Desinfektion werden in der Regel chemisch aggressive Reinigungsflüssigkeiten verwendet. Diese können insbesondere im Motorteil 20 Schäden anrichten wie beispielsweise Verschleiß an Lagern, negative Beeinflussung von Klebstoffen an Klebestellen innerhalb des Motorteils 20 und/oder Kontaktkorrosion. Aus diesen Gründen ist es vorteilhaft, wenn das Motorteil 20 von der Reinigung bzw. Desinfektion ausgenommen werden kann bzw. den Kontakt der Reinigungs- bzw. Desinfektionsmittel mit dem Motorteil 40 möglichst verhindert oder zumindest verringert werden kann.

Zu diesem Zwecke weist das Gebläse 1 einen Motorvorhof 50 auf. Der Motorvorhof 50 bietet viele Vorteile. Zum einen ist der Motorvorhof 50 eingerichtet und ausgebildet, um eine Kontamination des Motorteils 40 zu verhindern. Zum anderen kann über den Motorvorhof 50 verhindert oder vermieden werden, dass Reinigungs- und/oder Desinfektionsmittel an das Motorteil 40 gelangt. Die genannten Vorteile werden hierin nachfolgend detaillierter beschrieben.

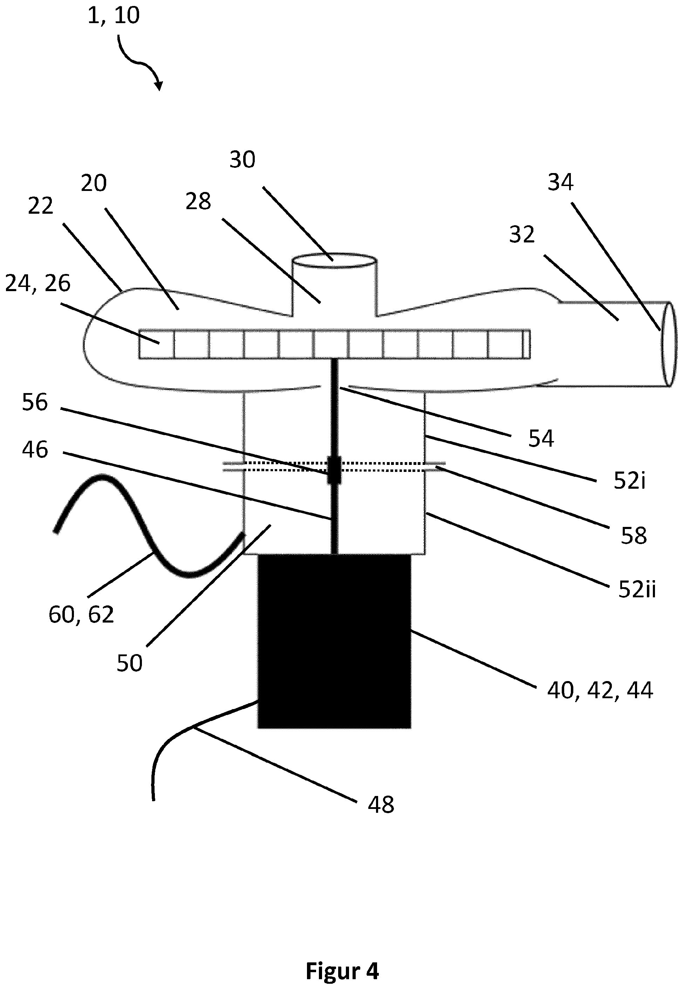

Der Motorvorhof 50 hat ein Vorhofgehäuse 52. Das Vorhofgehäuse 52 kann einteilig (siehe Fig. 1-3) oder zweiteilig (siehe Fig. 4) ausgebildet sein.

Der Vorhofgehäuse 52 ist zwischen dem Gebläsekopf 20 und dem Motorteil 40 angeordnet. Das Vorhofgehäuse 52 umschließt den Innenraum des Motorvorhofes 50. Zum einen ist das Vorhofgehäuse 52 im Wesentlichen luftdicht mit dem Lüfterradgehäuse 22 verbunden. Zum anderen ist das Vorhofgehäuse 52 im Wesentlichen luftdicht mit dem Motorgehäuse 42 verbunden. Die Antriebswelle 46 führt vom Motorteil 40 durch das Vorhofgehäuse 52 hindurch in den Gebläsekopf 20. Im Gebläsekopf 20 ist die Antriebswelle 46 mit dem Lüfterrad 24 verbunden.

Zwischen dem Motorvorhof 50 und dem Gebläsekopf 20 ist eine Durchführung 54 eingerichtet und angeordnet, durch die die Antriebswelle 46 des Motors 44 verläuft. Die Durchführung 54 kann mechanisch abgedichtet sein. Bevorzugt ist die Durchführung 54 nicht mechanisch abgedichtet, um Reibungsverluste zu vermeiden und besonders hohe Drehzahlen des Lüfterrades 24 zu erlangen. Die Durchführung 54 kann eine Leckage zwischen Gebläsekopf 20 und Motorvorhof 50 zulassen.

Das Gebläse 10 kann mindestens eine Zuführung 60 umfassen. Durch die Zuführung 60 kann der Motorvorhof 50 mit einem Fluid befüllt werden. Durch die Zuführung 60 kann der Motorvorhof 50 mit einem Druck P beaufschlagt werden. Durch die Zuführung 60 kann im Motorvorhof 50 auch eine Strömung erzeugt werden, die einen Druck P im Motorvorhof 50 erzeugt.

Bevorzugt wird Frischgas in den Motorvorhof 50 geleitet. Durch die Zuführung 60 kann Frischgas in das Vorhofgehäuse 52 geleitet werden, sodass der Motorvorhof 50 auf Überdruck gegenüber dem Gebläsekopf 20 gebracht werden kann. Der Überdruck im Motorvorhof 50 kann beispielsweise statisch auf einem Druckniveau gehalten werden. Der Überdruck im Motorvorhof 50 kann in manchen Ausführungsformen auch dynamisch an dem vom Gebläse 1 gelieferten Druck angepasst werden. Zu diesem Zweck können in der Zuführung 60 ein oder mehrere Zuführventile 62 angeordnet sein. Die Zuführventile 62 können beispielsweise ein Stufensystem darstellen, wobei durch die Zuführventile 62 verschiedene Druckstufen angeboten werden können. Alternativ oder zusätzlich kann auch ein zweites Gebläse 1' vorgesehen sein, das einen statischen und/oder dynamisch angepassten Druck P und/oder Fluss für die Zuführung 60 bereitstellen kann.

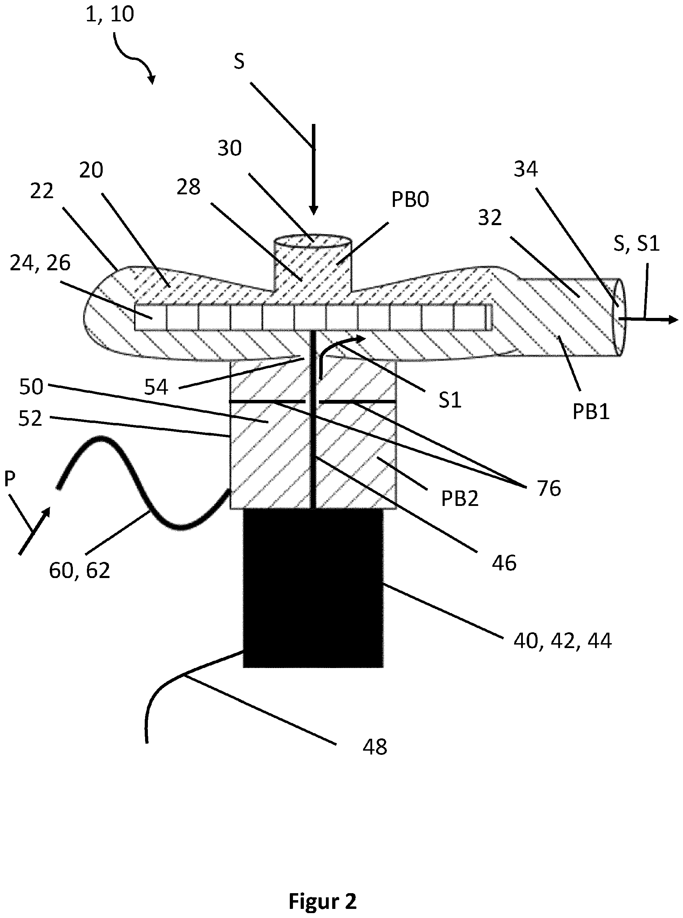

Die Zuführung 60 kann in dem Ausführungsbeispiel gemäß Fig. 1 oder Fig. 2 am Vorhofgehäuse 52 angeordnet sein. Das Vorhofgehäuse 52 weist zu diesem Zwecke eine Öffnung auf, an die die Zuführung 60 luftdicht gekoppelt werden kann. Über die Zuführung 60 kann das Frischgas zur Erzeugung des Überdrucks direkt in den Motorvorhof 50 eingeleitet werden.

Die Zuführung 60 kann an jeglichen geeignet erscheinenden Stellen des Vorhofgehäuses 52 angeordnet sein. Bevorzugt ist die Zuführung 60 benachbart zum Motorteil 40 angeordnet. Beispielsweise ist die Zuführung 60 in dem Bereich des Vorhofgehäuses 52 angeordnet, der sich maximal weit entfernt vom Gebläsekopf 20 befindet.

**Fig. 2** zeigt einen schematischen Ausschnitt aus dem Beatmungsgerät 1 mit dem Gebläse 10 im Querschnitt mit Darstellung verschiedener Strömungen S, S1 und unterschiedlicher Druckbereiche PB0, PB1, PB2.

In Fig. 2 sind die Druckverhältnisse innerhalb des Gebläses 1 schematisch und vereinfacht dargestellt. Unterschiedliche Bereiche innerhalb des Gebläses 1, in denen jeweils ein im Wesentlichen gleicher Druck P anliegt, sind unterschiedlich schraffiert dargestellt. Bei Betrieb des Gebläses 1 können im Wesentlichen drei Druckbereiche PB0, PB1 und PB2 vorliegen.

Im Saugstutzen 28 und im Bereich vor dem Lüfterrad 24 kann ein Druckbereich PB0 liegen, in dem ein Druck P0 anliegt.

In Strömungsrichtung nach dem Lüfterrad 24 in Richtung der Auslassöffnung 34 und im Druckstutzen 32 kann in einem Druckbereich PB1 ein durch das Lüfterrad 24 erzeugter, erhöhter Druck P1 anliegen. Der Druck P1 herrscht dementsprechend auch hinter dem Lüfterrad 24 im Bereich der Durchführung 54. Bei Betrieb des Gebläses 1 ist der Druck P1 größer als der Druck P0.

Im Motorvorhof 50 kann ein Druckbereich PB2 liegen, in dem ein Druck P2 anliegt. Der Druckbereich PB2 im Motorvorhof 50 wird über die Zuführung 60 auf den Druck P2 gebracht. Bevorzugt ist der Druck P2 größer als der Druck P1. Dadurch, dass der Druck P2 größer ist als der Druck P1, wird ein Eindringen der im Betrieb mit Keimen belasteten Atemluft verhindert. Im Gebläses 1 gilt demnach bevorzugt P2 > P1 > P0. Insbesondere gilt während des Betriebes des Gebläses 1 P2 > P1 > P0.

Durch den Überdruck im Motorvorhof 50 kann bevorzugt ein kleiner konstanter Luftstrom, eine zusätzliche Strömung S1, vom Motorvorhof 50 in den Gebläsekopf 20 entstehen.

Die zusätzliche Strömung S1 verhindert, dass Keime in den Motorvorhof 50 gelangen. Insbesondere verhindert die zusätzliche Strömung S1, dass Keime aus der Atemluft des Patienten, die in einem halbgeschlossenen Kreislauf in den Gebläsekopf 20 gelangt, vom Gebläsekopf 20 in den Motorvorhof 50 gelangen. Der Motorvorhof 50 kann somit als Reinraumschleuse ausgebildet und eingerichtet sein, was im Sinne der Erfindung bedeutet, dass der Motorvorhof 50 zumindest keimarm und bevorzugt keimfrei ist. Dadurch, dass der Motorvorhof 50 keimarm bzw. keimfrei ist, kann auch das Motorteil 40 keimarm bzw. keimfrei betreiben werden.

Keime umfassen im Sinne der Erfindung sämtliche luftgetragene Teilchen, die in dem Gebläse 1 unerwünscht sind und negative Auswirkungen auf den Patienten haben können. Keime umfassen insbesondere Mikroorganismen und/oder Kleinstlebewesen wie Pilze, Bakterien, Algen, Parasiten, Prionen, Protisten, Viren oder Viroiden sowie deren Derivate oder Vorstufen wie Pilzsporen, Sporen, Allergene, Toxine und dergleichen. Unter Keimen können insbesondere Krankheitserreger und/oder Pathogene verstanden werden.

Der Druck P2 wird in dieser Ausführungsform bevorzugt konstant gehalten. Durch die dargestellten Druckverhältnisse von P0, P1 und P2 und die Durchführung 54, die zwischen dem Lüfterradgehäuse 22 und dem Vorhofgehäuse 52 angeordnet ist, entsteht die zusätzliche Strömung S1. Die Strömung S1 verläuft aus dem Vorhofgehäuse 52 durch die Durchführung 54 in das Lüfterradgehäuse 22. Im Lüfterradgehäuse 22 kann sich die Strömung S1 mit der Luftströmung S vereinigen und zur Auslassöffnung 34 gefördert werden.

Über die Zuführung 60 wird bevorzugt Frischgas in den Motorvorhof 52 eingebracht, das auch für den Patienten bestimmt sein kann. Dadurch, dass zur Herstellung des Überdruckes im Motorvorhof 50 Frischgas verwendet wird, kann ein Teil des notwendigen Gasteintrages zur Auffrischung der Atemluft für den Patienten durch die beabsichtigte Leckage der Durchführung 54 erbracht werden.

Der Luftstrom der zusätzlichen Strömung S1 verhindert, dass Keime vom Gebläsekopf 20 in den Motorvorhof 50 gelangen können. Der Luftstrom der zusätzlichen Strömung S1 verhindert zudem, dass Keime an das Motorteil 40 gelangen können. Das Motorteil 40 und/oder der Motorvorhof 50 können somit nach einem Betrieb des Gebläses 1 mit einer Reinraumschleuse im Motorvorhof 50 von einer Reinigung und/oder Desinfektion ausgenommen werden.

Bei einer Reinigung und/oder Desinfektion kann das Gebläse 1 beispielsweise mit dem Motorteil 40 nach oben und mit dem Gebläsekopf 20 nach unten in ein Reinigungs- bzw. Desinfektionsgerät gegeben werden. Sodann kann über den Druckstutzen 32 und/oder den Saugstutzen 28 eine Reinigungsflüssigkeit in das Lüfterradgehäuse 22 eingeleitet werden und dieses bevorzugt vollständig füllen. Die Schwerkraft hält die Reinigungsflüssigkeit hierbei im Wesentlichen im unten liegenden Gebläsekopf 20. Potenziell kann über die Durchführung 54 dennoch Reinigungsflüssigkeit ungewollt in den Motorvorhof 50 gelangen. Damit die Reinigungsflüssigkeit nicht an das Motorteil 40 gelangen und dort Schäden verursachen kann, ist der Motorvorhof 50 zwischen dem Gebläsekopf 20 und dem Motorteil 40 angeordnet. Zusätzlich kann der Motorvorhof 50 spezielle Schutzmechanismen enthalten, die eingerichtet und ausgebildet sind, um die Reinigungsflüssigkeit vom Motorteil fernzuhalten.

Im Vorhofgehäuse 52 können in manchen Ausführungsformen beispielsweise ein oder mehrere Prallelemente 76 angeordnet sein (siehe Fig. 1 und Fig. 2). Die Prallelemente 76 sind eingerichtet und ausgebildet, um eindringende Reinigungsflüssigkeit in Form von Spritzwasser vom Motorteil 40 fernzuhalten. Die Prallelemente 76 können beispielsweise in Form einer oder mehrerer Blenden ausgebildet sein, die das Vorhofgehäuse in zwei Abschnitte unterteilen und nur im Bereich der Antriebswelle 46 einen möglichst geringen Freiraum belassen.

In manchen Ausführungsformen können alternativ oder zusätzlich im Vorhofgehäuse 52 ein oder mehrere Abflussöffnungen angeordnet sein (nicht gezeigt). Im Betrieb des Gebläses 1 können die Abflussöffnungen luftdicht verschlossen sein. Für einen Reinigungs- und/oder Desinfektionsprozess können die Abflussöffnungen hingegen geöffnet werden, sodass potenziell eindringende Reinigungsflüssigkeit aus dem Motorvorhof 50 abgeführt werden kann, bevor sie das Motorteil 40 erreicht. In manchen Ausführungsbeispielen können zu diesem Zwecke zusätzlich angeordnete Prallelemente 76 vorteilhaft eingerichtet und ausgebildet sein, um die eindringende Reinigungsflüssigkeit zu den Abflussöffnungen des Motorvorhofes 50 zu leiten.

In alternativen Ausführungsformen kann das Vorhofgehäuse 52 alternativ oder zusätzlich eine besondere Formgebung aufweisen, die potenziell eindringende Reinigungsflüssigkeit vom Motorteil 20 fernhalten kann (nicht gezeigt). Beispielsweise kann das Vorhofgehäuse 52 eine oder mehrere Einschnürungen aufweisen, sodass eine oder mehrere Verengungen entstehen, die das Spritzwasser der Reinigungsflüssigkeit vom Motorteil 40 fernhalten können. In einer beispielhaften Ausführungsform kann der Motorvorhof 50 eine sanduhrartige Formgebung aufweisen.

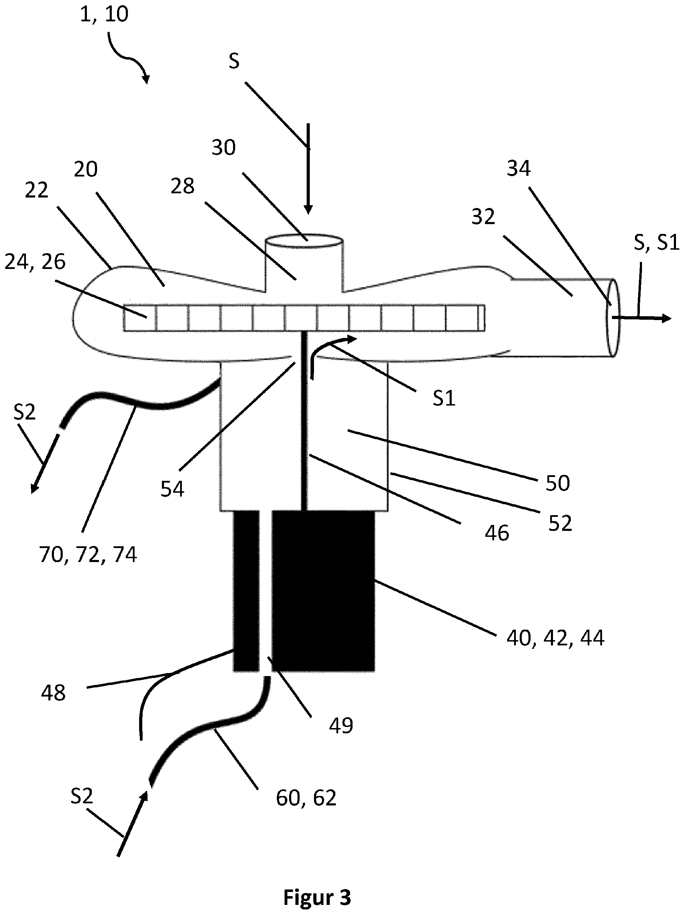

**Fig. 3** zeigt einen schematischen Ausschnitt aus einem alternativen Ausführungsbeispiel des Beatmungsgerätes 1 mit dem Gebläse 10 im Querschnitt.

Figur 3 zeigt, dass die Zuführung 60 auch am Motorteil 40 angeordnet sein kann. Das Motorteil 40 weist zu diesem Zwecke eine Öffnung auf, an die die Zuführung 60 luftdicht gekoppelt werden kann. Die Öffnung ist beispielsweise als mindestens ein Kanal 49 ausgebildet. Der mindestens eine Kanal 49 verbindet die Zuführung 60 mit dem Innenraum des Vorhofgehäuses 52. Es können auch mehrere Zuführungen 60 und/oder mehrere Kanäle 49 durch das Motorteil 40 vorgesehen sein. Über die Zuführung 60 kann Frischgas durch den Kanal 49 in das Vorhofgehäuse 52 eingeleitet werden.

Das Frischgas kann entsprechend dem vorstehend beschriebenen Ausführungsbeispiel verwendet werden, um einen konstanten Druck im Vorhofgehäuse 52 zu erzeugen.

Das über den Kanal 49 in das Vorhofgehäuse 52 eingeleitete Frischgas kann in diesem Ausführungsbeispiel bevorzugt auch dazu verwendet werden, eine weitere Strömung S2 zu erzeugen. Zu diesem Zwecke kann das Gebläse 1 in manchen Ausführungsformen eine Abführung 70 umfassen. Die Abführung 70 kann zudem ein oder mehrere Abführventile 72 und/oder Drosselelemente 74 aufweisen.

Die Abführung 70 ist beispielsweise eingerichtet und ausgebildet, um die Strömung S2 konstant durch den mindestens einen Kanal 49 und das Vorhofgehäuse 52 zu leiten. Die Strömung S2 kann auch dynamisch an die im Gebläsekopf 20 erzeugte (Atem-)Luftströmung S angepasst werden. Die Abführventile 72 und/oder Drosselelemente 74 in der Abführung 70 können hierbei derart eingerichtet sein, dass der Druck P2 im Motorvorhof 50 immer über dem Druck P1 im Gebläsekopf 20 liegt. Somit kann der Druck P2 dynamisch an die Druckverhältnisse im Gebläsekopf 20 angepasst werden.

Der Kanal 49 kann das Motorteil 40 in einer beispielhaften Ausführungsform der Länge nach gerade durchlaufen (siehe Fig. 3). In manchen Ausführungsformen ist es möglich, dass der Kanal 49 einen möglichst langen Weg im Motorteil 40 zurücklegt. Beispielsweise kann der Kanal 49 das Motorteil 40 schlaufenförmig durchlaufen und/oder ein oder mehrere Verzweigungen aufweisen. In manchen Ausführungsformen ist es auch denkbar, dass mehrere Kanäle 49 durch das Motorteil 49 hindurchführen (nicht gezeigt).

Der Kanal 49 bietet bzw. die mehreren Kanäle 49 bieten den zusätzlichen Vorteil, dass das Motorteil 40 bzw. der Motor 44 durch den Luftstrom des Frischgases gekühlt werden kann. Eine Kühlung des Motors 44 während des Betriebes ist üblicherweise notwendig. Durch die Ausbildung der Kanäle 49 können zusätzliche Kühlungsvorrichtungen des Motors 44 obsolet werden. Gleichzeitig bietet die Durchführung des Frischgases durch das Motorteil 40 den Vorteil, dass das Frischgas erwärmt werden kann. Erwärmtes Frischgas kann für eine Atemtherapie und/oder Beatmung vorteilhaft und/oder erforderlich sein.

Die Strömung S2 kann im Wesentlichen von der Zuführung 60 durch den Kanal 49 in das Vorhofgehäuse 52 strömen. Vom Vorhofgehäuse 52 kann die Strömung S2 im Wesentlichen durch die Abführung 70 abgeführt werden.

Durch die Strömung S2 kann im Motorvorhof 50 ein Überdruck erzeugt werden. Durch den Überdruck im Motorvorhof entsteht wiederum die zusätzliche Strömung S1, die vom Motorvorhof 50 in den Gebläsekopf 20 strömt. Die Strömung S1, die durch den Überdruck im Motorvorhof 50 entsteht, strömt durch die Durchführung 54 in das Lüfterradgehäuse 22. Im Lüfterradgehäuse 22 kann sich die Strömung S1 mit der Luftströmung S vereinigen und zur Auslassöffnung 34 gefördert werden.

Die Strömung S2, die durch die Abführung 70 abgeführt wird, kann vorteilhafterweise im halbgeschlossenen Kreislauf verbleiben. Sodann kann die durch die Abführung 70 abgeführte Strömung S2 beispielsweise wieder der Zuführung 60 zugeführt werden. Alternativ oder zusätzlich kann die durch die Abführung 70 abgeführte Strömung S2 auch über den Saugstutzen 28 in den Gebläsekopf 20 eingeleitet werden, wo sie zur Erzeugung der (Atem-)Luftströmung S zur Verfügung stehen kann.

**Fig. 4** zeigt einen schematischen Ausschnitt aus einem weiteren Ausführungsbeispiel des Beatmungsgerätes 1 mit dem Gebläse 10 im Querschnitt. Fig. 4 zeigt, dass der Motorvorhof 50 in manchen Ausführungsformen derart eingerichtet und ausgebildet sein kann, dass eine Trennung von Gebläsekopf 20 und Motor 40 möglich ist.

In manchen Ausführungsformen kann der Motorvorhof 50 lösbar an den Gebläsekopf 20 und/oder an das Motorteil 40 gekoppelt sein (nicht gezeigt). In einer vorteilhaften Ausführungsform kann das Vorhofgehäuse 52 auch zweigeteilt ausgebildet sein (siehe Fig. 4). Ein zweigeteilter Motorvorhof kann zwei Vorhofgehäuseteile 52i und 52 ii und eine Gehäusekopplung 58 umfassen.

Die Vorhofgehäuseteile 52i, 52ii können über die Gehäusekopplung 58 form- und/oder kraftschlüssig miteinander gekoppelt werden. Bevorzugt werden die Vorhofgehäuseteile 52i, 52ii luftdicht miteinander gekoppelt. Wenn die Vorhofgehäuseteile 52i, 52ii luftdicht miteinander gekoppelt sind, kann das Gebläse 1 in Betrieb genommen werden und eine (Atem-)Luftströmung S erzeugt werden.

Die Kopplung kann reversibel oder irreversibel sein. Bevorzugt ist die Kopplung reversibel ausgebildet. Eine reversible Kopplung der Vorhofgehäuseteile 52i, 52ii bietet den Vorteil, dass das Gebläse 1 getrennt werden kann. Eine Trennung des Gebläses 1 kann beispielsweise für eine Reinigung bzw. Desinfektion und/oder für eine andere Aufbereitung und/oder für eine Wartung bzw. zu Reparaturzwecken vorteilhaft sein.

Zum Zwecke der Trennung des Gebläses 1 kann die Antriebswelle 46 ebenfalls zweiteilig ausgeführt sein und eine Achsenkopplung 47 umfassen. Im Betrieb kann die Antriebswelle 46 über die Achsenkopplung 47 form- und/oder kraftschlüssig verbunden sein. Zur Trennung des Gebläses 1 kann die Achsenkopplung 47 gelöst werden. Die Achsenkopplung 47 kann an jeglichen Bereichen innerhalb der Antriebswelle 46 angeordnet sein. In dem konkreten Ausführungsbeispiel gemäß Fig. 4 ist die Achsenkopplung 47 in dem Bereich der Antriebswelle 46 angeordnet, der sich im Motorvorhof 50 befindet.

Beispielsweise kann das Gebläse 1 in Vorbereitung auf eine Reinigung und/oder Desinfektion getrennt werden. Auf diese Weise besteht die Möglichkeit, dass auf die Aufbereitung des Motorteils 20 verzichtet werden kann, da das Motorteil 20, wie oben beschrieben, im Betrieb keiner Kontamination ausgesetzt wurde. So kann das Motorteil 20 vor Beschädigungen durch einen Reinigungsprozess geschützt werden. Bei einer Trennung des Gebläses 1 kann sodann lediglich der kontaminierte Bereich, nämlich der Gebläsekopf 20 und/oder der Motorvorhof 50, gereinigt bzw. desinfiziert werden.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 1: Beatmungsgerät
- 10: Gebläse
- 20: Gebläsekopf
- 22: Lüfterradgehäuse
- 24: Lüfterrad
- 26: Schaufelelemente
- 28: Saugstutzen
- 30: Einlassöffnung
- 32: Druckstutzen
- 34: Auslassöffnung
- 36: Mittelachse
- 40: Motorteil
- 42: Motorgehäuse
- 44: Motor
- 46: Antriebswelle
- 47: Achsenkopplung
- 48: Elektrischer Anschluss
- 49: Kanal
- 50: Motorvorhof
- 52: Vorhofgehäuse
- 52i, 52ii: Vorhofgehäuseteile
- 54: Durchführung
- 58: Gehäusekopplung
- 60: Zuführung
- 62: Zuführventile
- 70: Abführung
- 72: Abführventile
- 74: Drosselelemente
- 76: Prallelemente

- P: Druck
- PB0, PB1, PB2: Druckbereiche
- S: (Luft-)Strömung
- S1: Zusätzliche Strömung
- S2: Weitere Strömung

## Patentansprüche

1. Beatmungsgerät (1), umfassend:
ein Gebläse (10) zum Erzeugen einer Luftströmung (S) im Gebläse (10), wobei das Gebläse (10) umfasst:
einen Gebläsekopf (20) mit einem drehbar gelagerten Lüfterrad (24);
mindestens ein Motorteil (40) mit wenigstens einem Motor (44) zum Betreiben des Gebläses (10), wobei der Motor (44) eine Antriebswelle (46) zum Antreiben des Lüfterrads (24) aufweist;
einen zwischen dem Motorteil (40) und dem Gebläsekopf (20) angeordneten Motorvorhof (50);
eine zwischen dem Motorvorhof (50) und dem Gebläsekopf (20) angeordnete Durchführung (54), wobei die Antriebswelle (46) vom Motor (44) durch den Motorvorhof (50) und die Durchführung (54) zum Lüfterrad (24) im Gebläsekopf (20) führt, wobei der Motorvorhof (50) im Wesentlichen luftdicht mit dem Gebläsekopf (20) verbunden ist, sodass eine Leckage zwischen dem Motorvorhof (50) und dem Gebläsekopf (20) lediglich über die Durchführung (54) möglich ist;
mindestens eine am Motorvorhof (50) und/oder am Motorteil (40) angeordnete Zuführung (60) zum Leiten eines Fluids in den Motorvorhof (50);
**dadurch gekennzeichnet, dass** das Gebläse (10) so ausgebildet ist, dass beim Erzeugen der Luftströmung (S) in mindestens einem ersten Druckbereich (PB1) im Gebläsekopf (20) ein erster Druck anliegt und in mindestens einem zweiten Druckbereich (PB2) im Motorvorhof (50) ein zweiter Druck anliegt, wobei der Motorvorhof (50) über die Zuführung (60) mit dem zweiten Druck beaufschlagt wird und der zweite Druck größer als der erste Druck ist, sodass eine Luftströmung in einer Richtung (S) hin zum Motor (44) verhindert wird.

2. Beatmungsgerät (1) nach Anspruch 1,
wobei das Gebläse (10) so ausgebildet ist, dass der zweite Druck konstant ist oder dynamisch an den ersten Druck angepasst werden kann.

3. Beatmungsgerät (1) nach einem der vorhergehenden Ansprüche,
wobei das Gebläse (10) ferner umfasst:
einen Saugstutzen (28) mit einer Einlassöffnung (30);
einen Druckstutzen (32) mit einer Auslassöffnung (34);
wobei das Gebläse (10) so ausgebildet ist, dass die Luftströmung (S) von der Einlassöffnung (30) zur Auslassöffnung (34) strömt und durch die unterschiedlichen Druckbereiche (PB1, PB2) mindestens eine zusätzliche Strömung (S1) vom Motorvorhof (50) durch die Durchführung (54) in den Gebläsekopf (20) erzeugt wird.

4. Beatmungsgerät (1) nach Anspruch 3,
wobei der Motorvorhof (50) so ausgebildet ist, dass er durch die zusätzliche Strömung (S1) als Reinraumschleuse fungiert, um zu verhindern, dass Keime vom Gebläsekopf (20) in den Motorvorhof (50) und/oder den Motor (44) eindringen, und so einen keimfreien Betrieb des Motors (44) zu ermöglichen.

5. Beatmungsgerät (1) nach einem der vorhergehenden Ansprüche,
wobei das Gebläse (10) ferner mindestens eine am Motorvorhof (50) und/oder am Motorteil (40) angeordnete Abführung (70) zum Ableiten des Fluids aus dem Motorvorhof (50) umfasst.

6. Beatmungsgerät (1) nach Anspruch 5,
wobei die Zuführung (60) und die Abführung (70) ausgebildet sind, um eine weitere Strömung (S2) zu erzeugen, indem das Fluid über die Zuführung (60) in den Motorvorhof (50) eingeleitet und über die Abführung (70) aus dem Motorvorhof (50) abgeleitet wird, wobei der Motorvorhof (50) über die weitere Strömung (S2) mit dem zweiten Druck beaufschlagt wird.

7. Beatmungsgerät (1) nach Anspruch 6,
wobei das Motorteil (40) mindestens einen Kanal (49) aufweist, an den die Zuführung (60) luftdicht gekoppelt ist, wobei der Kanal (49) in den Motorvorhof (50) mündet, wobei der Motorvorhof (50) über die Zuführung (60) und den Kanal (49) mit dem zweiten Druck beaufschlagt wird.

8. Beatmungsgerät (1) nach Anspruch 7,
wobei der Motorvorhof (50) die Abführung (70) umfasst und die weitere Strömung (S2) von der Zuführung (60) durch den Kanal (49) in den Motorvorhof (50) und von dort über die Abführung (70) strömt.

9. Beatmungsgerät (1) nach Anspruch 7 oder 8,
wobei der Kanal (49) ausgebildet ist, um die weitere Strömung (S2) derart zu leiten, dass die weitere Strömung (S2) den Motor (44) kühlt.

10. Beatmungsgerät (1) nach einem der vorhergehenden Ansprüche,
wobei das Gebläse (10) einteilig oder zweiteilig ausgebildet ist.

11. Beatmungsgerät (1) nach einem der vorhergehenden Ansprüche,
wobei der Motorvorhof (50) so ausgebildet ist, dass eine Trennung des Gebläsekopfes (20) vom Motorteil (40) möglich ist.

## Claims

1. A ventilator (1), comprising:
a fan (10) for generating an air flow (S) in the fan (10), wherein the fan (10) comprises:
a fan head (20) with a rotatably mounted fan wheel (24);
at least one motor part (40) with at least one motor (44) for operating the fan (10), wherein the motor (44) has a drive shaft (46) for driving the fan wheel (24);
a motor pre-chamber (50) arranged between the motor part (40) and the fan head (20);
a passage (54) arranged between the motor pre-chamber (50) and the fan head (20), wherein the drive shaft (46) leads from the motor (44) through the motor pre-chamber (50) and the passage (54) to the fan wheel (24) in the fan head (20), wherein the motor pre-chamber (50) is connected in a substantially air-tight manner to the fan head (20), such that a leak between the motor pre-chamber (50) and the fan head (20) is only possible via the passage (54);
at least one feed line (60) arranged on the motor pre-chamber (50) and/or on the motor part (40) for conducting a fluid into the motor pre-chamber (50);
**characterized in that** the fan (10) is designed such that, when the air flow (S) is generated, a first pressure is present in at least one first pressure region (PB1) in the fan head (20) and a second pressure is present in at least one second pressure region (PB2) in the motor pre-chamber (50), wherein the second pressure is applied to the motor pre-chamber (50) via the feed line (60) and the second pressure is greater than the first pressure, such that an air flow in a direction (S) towards the motor (44) is prevented.

2. The ventilator (1) according to claim 1,
wherein the fan (10) is designed such that the second pressure is constant or can be dynamically adapted to the first pressure.

3. The ventilator (1) according to any one of the preceding claims,
wherein the fan (10) also comprises:
an intake port (28) with an inlet opening (30);
a discharge port (32) with an outlet opening (34);
wherein the fan (10) is designed such that the air flow (S) flows from the inlet opening (30) to the outlet opening (34) and, due to the different pressure regions (PB1, PB2), at least one additional flow (S1) from the motor pre-chamber (50) through the passage (54) and into the fan head (20) is generated.

4. The ventilator (1) according to claim 3,
wherein the motor pre-chamber (50) is designed such, due to the additional flow (S1), it functions as a clean room airlock in order to prevent germs from the fan head (20) from entering the motor pre-chamber (50) and/or the motor (44) and thus to enable germ-free operation of the motor (44).

5. The ventilator (1) according to any one of the preceding claims,
wherein the fan (10) also comprises at least one discharge line (70) arranged on the motor pre-chamber (50) and/or on the motor part (40) for discharging the fluid from the motor pre-chamber (50).

6. The ventilator (1) according to claim 5,
wherein the feed line (60) and the discharge line (70) are designed to generate a further flow (S2) in that the fluid is introduced into the motor pre-chamber (50) via the feed line (60) and discharged from the motor pre-chamber (50) via the discharge line (70), wherein the second pressure is applied to the motor pre-chamber (50) via the further flow (S2).

7. The ventilator (1) according to claim 6,
wherein the motor part (40) has at least one channel (49), to which the feed line (60) is coupled in an air-tight manner, wherein the channel (49) opens into the motor pre-chamber (50), wherein the second pressure is applied to the motor pre-chamber (50) via the feed line (60) and the channel (49).

8. The ventilator (1) according to claim 7,
wherein the motor pre-chamber (50) comprises the discharge line (70) and the further flow (S2) flows from the feed line (60) through the channel (49) into the motor pre-chamber (50) and from there via the discharge line (70).

9. The ventilator (1) according to claim 7 or 8,
wherein the channel (49) is designed to conduct the further flow (S2) such that the further flow (S2) cools the motor (44).

10. The ventilator (1) according to any one of the preceding claims,
wherein the fan (10) is designed in one piece or in two pieces.

11. The ventilator (1) according to any one of the preceding claims,
wherein the motor pre-chamber (50) is designed such that it is possible to separate the fan head (20) from the motor part (40).

## Revendications

1. Appareil respiratoire (1), comportant :
un ventilateur (10) destiné à produire un flux d'air (S) dans le ventilateur (10), dans lequel le ventilateur (10) comporte :
une tête de ventilateur (20) dotée d'une roue de ventilation (24) ;
au moins une partie de moteur (40) dotée d'au moins un moteur (44) pour le fonctionnement du ventilateur (10), dans lequel le moteur (44) présente un arbre d'entraînement (46) pour l'entraînement de la roue de ventilation (24) ;
une préchambre de moteur (50) disposée entre la partie de moteur (40) et la tête de ventilateur (20) ;
un passage (54) disposé entre la préchambre de moteur (50) et la tête de ventilateur (20), dans lequel l'arbre d'entraînement (46) mène du moteur (44) à la roue de ventilation (24) dans la tête de ventilateur (20) à travers la préchambre de moteur (50) et le passage (54), dans lequel la préchambre de moteur (50) est reliée à la tête de ventilateur (20) de façon essentiellement étanche à l'air, de telle façon qu'une fuite entre la préchambre de moteur (50) et la tête de ventilateur (20) est possible seulement par le biais du passage (54) ;
au moins une alimentation (60) disposée sur la préchambre de moteur (50) et/ou sur la partie de moteur (40) pour l'acheminement d'un fluide dans la préchambre de moteur (50) ;
**caractérisé en ce que** le ventilateur (10) est conçu de telle façon que lors de la production du flux d'air (S), une première pression est appliquée dans au moins une première région de pression (PB1) dans la tête de ventilateur (20) et une deuxième pression est appliquée dans au moins une deuxième région de pression (PB2) dans la préchambre de moteur (50), dans lequel la préchambre de moteur (50) est sollicitée avec la deuxième pression par le biais de l'alimentation (60) et la deuxième pression est supérieure à la première pression, de sorte qu'un flux d'air dans une direction (S) vers le moteur (44) est empêché.

2. Appareil respiratoire (1) selon la revendication 1,
dans lequel le ventilateur (10) est conçu de telle façon que la deuxième pression est constante ou peut être adaptée de manière dynamique à la première pression.

3. Appareil respiratoire (1) selon l'une des revendications précédentes,
dans lequel le ventilateur (10) comporte en outre :
une tubulure d'aspiration (28) avec une ouverture d'admission (30) ;
une tubulure de pression (32) avec une ouverture d'échappement (34) ;
dans lequel le ventilateur (10) est conçu de telle façon que le flux d'air (S) s'écoule de l'ouverture d'admission (30) vers l'ouverture d'échappement (34) et au moins un écoulement supplémentaire (S1) est produit par les différentes régions de pression (PB1, PB2) à partir de la préchambre de moteur (50) à travers le passage (54) vers la tête de ventilateur (20).

4. Appareil respiratoire (1) selon la revendication 3,
dans lequel la préchambre de moteur (50) est conçue de manière à fonctionner comme un sas de chambre propre de par l'écoulement supplémentaire (S1), pour empêcher l'introduction de germes de la tête de ventilateur (20) dans la préchambre de moteur (50) et/ou le moteur (44), afin de permettre un fonctionnement stérile du moteur (44).

5. Appareil respiratoire (1) selon l'une des revendications précédentes,
dans lequel le ventilateur (10) comporte en outre au moins une évacuation (70) disposée sur la préchambre de moteur (50) et/ou sur la partie de moteur (40) pour le drainage du fluide hors de la préchambre de moteur (50).

6. Appareil respiratoire (1) selon la revendication 5,
dans lequel l'alimentation (60) et l'évacuation (70) sont conçues pour produire un écoulement supplémentaire (S2), dans lequel le fluide est introduit dans la préchambre de moteur (50) par le biais de l'alimentation (60) et drainé hors de la préchambre de moteur (50) par le biais de l'évacuation (70), dans lequel la préchambre de moteur (50) est sollicitée avec la deuxième pression par le biais de l'écoulement supplémentaire (S2).

7. Appareil respiratoire (1) selon la revendication 6,
dans lequel la partie de moteur (40) présente au moins un canal (49), auquel l'alimentation (60) est accouplée de façon étanche à l'air, dans lequel le canal (49) débouche dans la préchambre de moteur (50), dans lequel la préchambre de moteur (50) est sollicitée avec la deuxième pression par le biais de l'alimentation (60) et du canal (49).

8. Appareil respiratoire (1) selon la revendication 7,
dans lequel la préchambre de moteur (50) comporte l'évacuation (70) et l'écoulement supplémentaire (S2) s'écoule à partir de l'alimentation (60) à travers le canal (49) vers la préchambre de moteur (50) et de là par le biais de l'évacuation (70).

9. Appareil respiratoire (1) selon la revendication 7 ou 8,
dans lequel le canal (49) est conçu pour acheminer l'écoulement supplémentaire (S2) de telle façon que l'écoulement supplémentaire (S2) refroidit le moteur (44).

10. Appareil respiratoire (1) selon l'une des revendications précédentes,
dans lequel le ventilateur (10) est conçu en une partie ou en deux parties.

11. Appareil respiratoire (1) selon l'une des revendications précédentes,
dans lequel la préchambre de moteur (50) est conçue de manière à permettre une séparation de la tête de ventilateur (20) d'avec la partie de moteur (40).
